Europäisches Patentamt

European Patent Office

Office européen des brevets

(11) Publication number: **0 085 033**
A1

## (12) EUROPEAN PATENT APPLICATION

(21) Application number: 83850004.9

(22) Date of filing: 14.01.83

(51) Int. Cl.³: **A 61 K 31/66**, A 61 K 31/70
// (A61K31/70, 31/66)

(30) Priority: 18.01.82 SE 8200252

(43) Date of publication of application: 03.08.83
**Bulletin 83/31**

(84) Designated Contracting States: **AT BE CH DE FR GB IT
LI LU NL SE**

(71) Applicant: **PHARMACIA AKTIEBOLAG, Rapsgatan 7,
S-754 50 Uppsala (SE)**

(72) Inventor: **Hultman, Jan Lennart, Porsvägen 7,
S-752 46 Uppsala (SE)**
Inventor: **Ronquist, Gunnar, Dalbovägen 35 A,
S-752 52 Uppsala (SE)**

(74) Representative: **Fogelberg, Lennart et al, STENHAGEN
PATENTBYRA AB Karlavägen 18, S-114 31 Stockholm
(SE)**

(54) A pharmaceutical composition.

(57) The invention relates to a pharmaceutical composition for preventing and treating ischemic cell damage. The composition contains a physiologically acceptable, water-soluble salt of phosphoenolpyruvic acid and a physiologically acceptable, water-soluble salt of adenosine-5'-triphosphoric acid in a mutual ratio from 5:1 to 1000:1, preferably from 10:1 to 500:1, calculated on a molar basis.

EP 0 085 033 A1

0085033

# A PHARMACEUTICAL COMPOSITION

The present invention relates to a pharmaceutical composition. More specificly, the invention relates to a pharmaceutical composition for preventing and treating ischemic cell damage. The invention also relates to a method for preventing and treating ischemic cell damage in mammals, including humans, or in organs removed therefrom.

Ischemic damage to the body cells of a mammal occurs when the flow of blood in a part of the body is impeded to such an extent that the supply of oxygen to the cells of said part of the body ceases or is greatly reduced. The reason why the cell requires oxygen is because the predominant part (about 92 %) of adenosine-5'-triphosphate (ATP), necessary to the survival of the cell, is formed by a mechanism which involves an aerobic oxidation. In the event of an oxygen deficiency, the ATP-content of the cell falls, the resultant injury to the cell being initially reversible, i.e. the damage can be stopped when the ATP-content of the cell returns to more normal values, and in time becomes irreversible, resulting in the death of the cell and subsequently (with an increase in the number of dead cells) the death of the organ.

In order to reduce the extent of ischemic cell damage, it is feasible to think that the ATP-content of the cells could be increased by supplying ATP directly to the cells, thereby to avoid the aerobic forming reaction for ATP. It has been found, however, that exogenous ATP cannot penetrate into the cells to the extent required to markedly ease the ischemic damage.

In accordance with the present invention it has now surprisingly been found that an introduction of relatively small quantities of ATP enables another substance, namely a salt of phosphoenolpyruvic acid, to be introduced into the cell in larger quantities simultaneously with ATP, where said substance then contributes to the conversion of adenosine diphosphate (ADP) present in the cell to ATP in one single reaction step which does not require the presence of oxygen.

Accordingly, the present invention relates to a pharmaceutical composition for preventing and treating ischemic cell damage, said composition being characterized in that it includes a physiologically acceptable, water-soluble salt of phosphoenolpyruvic acid (which salt is hereinafter referred to as PEP) and a physiologically acceptable, water-soluble salt of adenosine-5'-triphosphoric acid (which salt is hereinafter referred to as ATP) in a mutual ratio of 5:1 to 1000:1, preferably from 10:1 to 500:1, calculated on a molar basis.

As examples of physiologically acceptable salts of the two compounds can be mentioned sodium salts, magnesium salts or ammonium salts and salts with physiologically acceptable organic amines, such as tris(hydroxymethyl)aminomethane (called tris), methyl glucamine, arginine, ornitine or histidine.

Conveniently, the molar ratio between PEP and ATP can, for example, be greater than 20:1, for example greater than 50:1, and can be lower than 400:1, e.g. lower than 300:1, e.g. lower than 200:1.

With the composition according to the invention there is obtained with the two incorporated components a synergistic effect. This effect cannot be obtained with solely one of the components. Neither can such an advantageous effect, free from complications, be obtained with PEP in combination with other additives.

In addition to the salts of phosphoenolpyruvic acid and of adenosine-5'-triphosphoric acid the pharmaceutical composition according to the invention comprises agents for establishing physiological conditions and, when necessary, water as a carrier.

For the purpose of administration, the composition according to the invention is in the form of a sterile solution for parenteral administration and organ perfusion, but is preferably stored in a freeze-dried form because of the unfavourable stability properties of the solutions.

Agents for establishing physiological conditions are, for instance, buffer substances, ions for establishing correct osmolality and, when necessary, colloid-osmotically active substances, such as albumin and clinical dextrans, e.g. Dextran 40.

The composition according to the invention can be prepared, for example, by dissolving PEP and ATP (e.g. in freeze-dried form) in the aforementioned mutual ratios in a physiologically acceptable aqueous liquid, e.g. in a physiological saline solution, whereafter the pH of the solution is adjusted as necessary with the aid of a base or an acid (which will provide physiologically acceptable salts) to obtain a physiologically acceptable pH (which is selected in dependence upon quantity and area of application, for example within the pH-range of 3 - 7.6, such as 4 - 7). Subsequent to being sterilized, e.g. by sterile filtration, the resultant solution can either be used directly or freeze-dried to a solid substance, which when subsequently used is dissolved in a physiologically acceptable aqueous liquid, e.g. a physiologically acceptable salt solution or buffer solution, such as a phosphate buffer solution or an infusion solution of conventional composition.

The pH can be adjusted with the use of bases or acids which provide physiologically acceptable salts, e.g. inorganic bases, preferably alkaline metal hydroxides, such as sodium hydroxide or potassium hydroxide, or organic bases, e.g. basic amino acids, such as arginine or ornitine or tris or methylglucamine or histidine or inorganic acids, such as HCl or organic acids, such as acetic acid.

The concentrations of the solutions of the two components may vary within wide limits, depending upon the area of application (e.g. perfusion or directed infusion, type of organ etc.) and upon whether it is elected to supply the components in a greater or smaller volume. For example, the concentration of PEP in the solution can accordingly vary within the range of 10 to 3000 mmoles per litre, such as 20 to 2000 mmoles per litre, the concentration of ATP being selected in relation hereto in accordance with the aforegiven molar stoichiometric relationships.

In accordance with a further method of preparation, a sterile PEP and ATP in solid state (e.g. in freeze-dried form) are dry-mixed in the aforegiven mutual quantity ratio, whereafter the mixture is conveniently pre-packed in dosage units, such as bottles containing a quantity of the mixture corresponding to a normal dose. Prior to being used, a dosage quantity of the mixture is then dissolved in a given volume of a sterile, physiologically acceptable aqueous liquid, e.g. an isotonic buffer solution, such as a tris buffer solution or a phosphate buffer solution.

In accordance with one embodiment of the invention, the composition may also contain magnesium ions, for example in a stoichiometric relationship of about 1:1 in relation to ATP. These ions may be supplied, for example, by introducing a physiologically acceptable magnesium salt, preferably $MgCl_2$, in a measured quantity at a suitable stage in the preparation process, e.g. added to a buffer solution in which a powderous mixture of PEP and ATP is dissolved, or by dissolving the magnesium salt in a physiologically iso- tonic saline solution in conjunction with dissolving PEP and ATP therein.

The composition according to the invention is administered in the form of a sterile solution for parenteral administration and organ perfusion, to prevent and treat ischemic cell damage (preferably in humans but also in other mammals). An important area of application for the composition according to the invention is its use as a perfusion and preservation solution for organs, e.g. in open heart surgery, and organ transplantations. Another important area of application is the use of the composition as an infusion solution for directed infusion through cathethers for treating ischemic brain and heart damage as result of heart failure, drowning, an overdose of certain types of pharmaceutical preparations, such as soporifics etc.

The active substance concentration of the solutions intended for administration varies in dependence upon the distance from the site at which the solutions are administered to the location of the ischemic cell damage to be treated. Suitably, the concentration of the solution at the location of application remote from the location of the damage is such as to achieve an extracellular concentration of ATP at the injured location of an order of magnitude of 1 - 4 %, preferably 2 - 3 %, of the intracellular physiological concentration of ATP in the body cells. This means, for example, in the case of perfusion solutions and preservation solutions that the concentration of such solutions with respect to PEP should be such that the organ (e.g. a heart) obtains during the whole of the perfusion period a total of, for example, 20 - 60 mmoles PEP. If the total perfusion volume is calculated to be one litre, the solution may contain for example 20 - 60 mmoles PEP per litre. If the solution volume totals two litres, the solution may correspondingly contain 10 - 30 mmoles PEP per litre. The amount of ATP is selected in relation to the amount of PEP, so as to obtain the aforegiven mutual molar relationship.

When treating ischemic cell damage in organs by infusion, the dosage may be, for example, 0.1-2, e.g. 0.5-1, mmole PEP per kilo body weight, the amount of ATP being selected in relation to the amount of PEP so as to fulfil the aforegiven molar stoichiometry. For example, in the case of a directed dosing of PEP of 1 mmole/kilo body weight to a patient weighing 75 kilos, said patient obtains a total of 75 mmoles PEP (and ATP in relation thereto). This can be administered with a 50 ml solution, the PEP concentration of which is 1500 mmoles per litre. With respect to infusion solutions for directed infusion through catheters, a higher concentration is suitably selected, to compensate for dilution and losses from the infusion site to the target organ.

Thus, the amounts in which the composition according to the invention is administered will vary with the area of application.

The invention also relates to a method of preventing and treating ischemic cell damage in mammals, including humans, or in organs removed therefrom, the method being characterized by supplying to the mammal or to the organ a therapeutically effective dose of a pharmaceutical composition according to the invention.

The invention will now be desribed in more detail with reference to a number of examples.

## EXAMPLE 1

1.6 mmole monosodium phosphoenolpyruvate and 0.01 mmole disodiumadenosine-5'-triphosphate are dissolved, while stirring, in 100 ml of a physiological saline solution (0.9 g NaCl/ 100 ml), whereafter the pH of the solution is adjusted to 6.4 - 7.0 by adding dropwise to said solution 1 N and 0.1 N sodium hydroxide solution. The resultant solution is sterilized by sterile filtration, poured into 20 ml bottles and lyophilized, whereafter the bottles are sealed (all under aseptic conditions).

Subsequent to dissolving the content of one bottle in a physiological aqueous solution, the resultant composition is suited for organ perfusion in experiments on rats.

## EXAMPLE 2

The procedure described in Example 1 is followed but with 400 mmoles of monosodium phosphoenolpyruvate and 4 mmoles of disodium-adenosine-5'-triphosphate.

Subsequent to dissolving the contents of one bottle in a physiological aqueous solution, the resultant composition is suitable for human use with directed infusion.

## PHARMACOLOGICAL EXPERIMENTS

For these experiments there was used a model comprising an excised rat heart, substantially in accordance with Gamble et al., Am. J. Physiol. 219 : 604612 (1970) although modified so that the abdominal aorta of the supporting rat was connected directly to the ascending aorta of the excised heart in accordance with the following:

Unstarved male Sprague-Dawley-rats weighing about 300 g were used in the experiment.

The ascending aorta of an excised rat heart was connected to the abdominal aorta of a living rat ("supporting rat") functioning as a heart-lung-machine for the excised rat heart by means of a tube ("pump tube"). The coronary effluent from the excised heart was collected in a tempered water-jacketed funnel and retransfused to the supporting rat. After the ischemic period, a pulsatile blood flow was established from the supporting rat to the excised heart. The temperature of the supporting rat and the excised rat heart was kept at $37^{\circ} \pm 1^{\circ}C$.

Rats intended for supporting function were anaesthetized by intraperitoneal administration of 5-sec.-butyl-5-ethyl-2-thiobarbituric acid (Inactin ® from Byk-Gulden Lomberg Chem. Fabrik GmbH, Konstanz, West Germany) 120 mg per kilo body weight. After tracheostomy the right carotis communis was cannulated for continuous blood pressure recording by an EMT 34 transducer (from Siemens-Elema AB, Solna, Sweden) and an M 34 recorder (from Siemens-Elema AB, Solna, Sweden). The right jugular vein was cannulated for transfusion. Heparin in a dose of 200 IU (international units) was administred intravenously. The supporting rat breathed air spontaneously.

The abdominal aorta was cannulated and the cannula was fixed to the aorta by silk ligatures. The cannula was cut close to the aorta and rejoined by a more flexible piece of silicone rubber tubing, to permit easy clamping. The connections permitted blood sampling and infusion. The body temperature was recorded continuously in the abdominal cavity.

The heart donors wer anaesthetized with ether. After 200 IU of heparin intravenously the blood was rapidly collected from the abdominal aorta. Subsequent thoracotomy and ligation of the aortic arch branches, the heart-arch-preparation was excised. The time required for this procedure was 3 - 5 minutes.

The ascending aorta was attached to the cannula from the supporting rat. Electrodes for ECG-recording were placed at the apex, the right atrium and the aortic arch. During the ischemic period, the heart was kept in a water-jacketed funnel filled with plain saline solution at 37 - 38°C.

The retransfusion system consisted of a peristaltic roller pump (Multiperplex 2115 from LKB-Produkter AB, Bromma, Sweden), a temperature-controlled water bath and an air-bubble trap. All connections were of silicone rubber tubing. The system was primed with 20 ml of heparinized blood collected from the heart donor and an additional rat. The retransfusion of blood was adjusted to constant blood-pressure of the supporting rat by regulating the height of the bubble trap.

Normothermic ischemia was promoted by clamping the pump tube. After the ischemic period, the tube clamp was released and a pulsative blood-flow was re-established through the coronary arteries of the excised heart.

All excised hearts were subjected to 15 minutes of complete global (37°C) ischemia, calculated from the time from excision of the heart to the start of reperfusion. Reperfusion with the pulsative arterial blood flow from the supporting rat was continued for 30 minutes, after which all hearts were immediately freeze-clamped with a pair of chilled tongues (in liquid nitrogen) and transferred in liquid nitrogen. Freezing was instantaneous.

There was administred to a group of 11 excised rat hearts (group 1) through continuous perfusion simultaneously with the start of the reperfusion, 10 ml of physiological saline solution at room temperature through the pump tube. Perfusion was continued for seven minutes.

10 ml of solution according to Example 1 were administred to a further group of seven excised rat hearts (group 2) in the same manner as in group 1, by perfusion through the pump tube.

The following biochemical analyses were made:

Subsequent to being frozen, the excised hearts were stored at a temperature of $-70^{\circ}$C and then freeze-dried at a temperature beneath $-4^{\circ}$C. Adenine-nucleotides and energy charge were determined after perchloric acid extraction in the heart material using an HPLC-method (HPLC = High Performance Liquid Chromatography). Thus, the analyses made it possible to accurately determine quantitatively the adenosine triphosphate (ATP), adenosine-diphosphate (ADP) and adenosine-monophosphate (AMP) enabling subsequent calculation of the energy charge (ACP, i.e. "adenylate charge potential") in accordance with the Atkinson equation (see Atkinson, D.E.: "The energy charge of the adenylate pool as a regulatory parameter. Interaction with feedback modifiers". Biochemistry 7 (1968) 4030-4038). The enzyme creatine kinase (CK) efflux was followed as an indicator of tissue damage. The principle for determining CK in serum was in accordance with recommendations from the Scandinavian Enzyme Committee, and the enzymatic activity was expressed in $\mu$kat/l. (With respect to the HPLC-method applied, references were made to Hartwick, R.A. and Brown, P.R., "The performance of microparticle chemically bonded anion-exchange resins in the analysis of nucleotides.", J. Chromatog. 112 (1979) 651 - 660).

The results are given in the following table.

| Heart group | ACP | Total adenine-nucleotide content $\mu$mol/g dry weight | ATP/ADP ratio | Creatine kinase ($\mu$kat/l) after reperfusion in minutes | | | |
|---|---|---|---|---|---|---|---|
| | | | | 0 | 10 | 20 | 30 |
| 1 (n=11) | 0.85±0.06 | 12.59±2.94 | 4.90±1.40 | 8.3 ±4.0 | 18.3 ±5.2 | 30.2 ±8.6 | 34.8 ±9.8 |
| 2 (n=7) | 0.91±0.01 | 14.73±1.75 | 6.21±0.74 | 8.7 ±2.7 | 15.4 ±3.5 | 21.1 ±4.7 | 23.4 ±4.3 |

n = the number of hearts in the group.
The values are given as mean values ± standard deviations.

When making a comparison between group 1 (control group) and group 2 (treatment group) several important differences were found :

The difference in ACP is statistically significant ($p < 0.02$) as are also the differences in ATP/ADP-ratio ($p < 0.05$) and the difference in the release of creatine kinase (as a measurement of cell-leakage) in the range of 20 - 30 minutes ($p < 0.02$).

In addition to the important biochemical differences shown in the table, the treated hearts in group 2 exhibited substantially improved contractibility when loaded than those in group 1, which had not been treated with the solution according to Example 1.

CLAIMS

1. A pharmaceutical composition for preventing and treating ischemic cell damage, c h a r a c t e r i z e d i n that it contains a physiologically acceptable, water-soluble salt of phosphoenolpyruvic acid and a physiologically acceptable, water-soluble salt of adenosine-5'-triphosphoric acid in a mutual ratio of from 5:1 to 1000:1, preferably from 10:1 to 500:1, calculated on a molar basis.

2. A pharmaceutical composition according to Claim 1, c h a r a c t e r i z e d i n that it in addition to the salts of phosphoenolpyruvic acid and of a adenosine-5'-triphosphoric acid comprises agents for establishing physiological conditions and, when necessary, water as a carrier.

3. A pharmaceutical composition according to Claim 1, c h a r a c t e r i z e d i n that it exists in a freeze-dried form or in the form of a sterile solution for parenteral administration.

4. A pharmaceutical composition according to Claim 1, c h a r a c t e r i z e d i n that it contains magnesium ions in a stoichiometric relationship of about 1:1 in relation to adenosine triphosphate.

5. A method of preventing and treating ischemic cell damage in mammals, including humans, or in organs excised therefrom, c h a r a c t e r i z e d b y administering to the mammal or to said organ a therapeutically effective dose of a pharmaceutical composition according to Claim 1.

6. A process for the preparation of a pharmaceutical composition for preventing and treating ischemic cell damage, c h a r a c t e r i z e d b y dissolving a physiologically acceptable water-soluble salt of phosphoenolpyruvic acid and a physiologically acceptable, water-soluble salt of adenosine-5'-triphosphoric acid in a mutual ratio of from 5:1 to 1000:1, preferably from 10:1 to 500:1, calculated on a molar basis, in a physiologically acceptable aqueous liquid, adjusting the pH of the solution as necessary with the aid of a base or an acid to obtain a physiologically acceptable pH, then sterilizing the solution and, when desired, freeze-drying the resultant solution.

# PARTIAL EUROPEAN SEARCH REPORT
which under Rule 45 of the European Patent Convention shall be considered, for the purposes of subsequent proceedings, as the European search report

European Patent Office

Application number

| DOCUMENTS CONSIDERED TO BE RELEVANT | | | EP 83 85 0004 |
|---|---|---|---|
| Category | Citation of document with indication, where appropriate, of relevant passages | Relevant to claim | CLASSIFICATION OF THE APPLICATION (Int. Cl. 3) |
| A | FR - M - 3 246M (PHARMACODYNAMIE ET METABOLISME)<br><br>* page 3; abstract * | 1-4,6 | A 61 K 31/66<br>31/70<br>//(A 61 K 31/70<br>31/66) |
| A | US - A - 4 216 211 (M.D. FRANCIS) | | |

TECHNICAL FIELDS SEARCHED (Int. Cl. 3)

A 61 K
C 07 F

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-4,6

Claims searched incompletely:

Claims not searched: 5

Reason for the limitation of the search:

Method for treatment of the human or animal body by surgery or therapy (see article 52(4) of the European Patent Convention)

| Place of search | Date of completion of the search | Examiner |
|---|---|---|
| The Hague | 15-04-1983 | BRINKMANN |